# EUROPEAN PATENT SPECIFICATION

(11) **EP 4 512 408 B1**
(45) Date of publication and mention of the grant of the patent: **15.07.2026**
(21) Application number: 23792176.2
(22) Date of filing: 19.04.2023
(51) Int. Cl.: A61K 36/232, A61K 36/233, A61K 36/53, A61K 36/484, A61K 36/71, A61K 36/70, A61P 31/14, A23L 33/105, A61K 8/9789, A61Q 19/00, A61K 36/539, A61K 36/708, A23K 10/30

(54) **COMPOSITION, FOR PREVENTING, AMELIORATING, OR TREATING CORONAVIRUS INFECTIONS, COMPRISING HERBAL MEDICINE EXTRACT AS ACTIVE INGREDIENT**
ZUSAMMENSETZUNG ZUR PRÄVENTION, LINDERUNG ODER BEHANDLUNG VON CORONAVIRUS-INFEKTIONEN MIT EINEM KRÄUTERMEDIZINEXTRAKT ALS WIRKSTOFF
COMPOSITION POUR LA PRÉVENTION, L'ATTÉNUATION OU LE TRAITEMENT D'UNE INFECTION À CORONAVIRUS, COMPRENANT UN EXTRAIT DE COMPLEXE D'HERBES MÉDICINALES EN TANT QUE PRINCIPE ACTIF

(30) Priority: 20.04.2022 KR 20220048909
(43) Date of publication of application: 26.02.2025
(73) Proprietor: Korea Institute of Oriental Medicine, Daejeon 34054 (KR)
(72) Inventor: KWON, Sunoh, Daejeon 34504 (KR); JIN, Young-Hee, Daejeon 34054 (KR); LEE, Sang-Myeong, Cheongju-si Chungcheongbuk-do 28644 (KR); KIM, Sanghyun, Daejeon 34054 (KR); YOON, Jiwon, Daejeon 34054 (KR)
(74) Representative: Ipsilon
(86) International application number: PCT/KR2023/005307
(87) International publication number: WO 2023/204602

(56) References cited:
- CN-A- 107 802 752
- CN-A- 110 433 275
- CN-A- 112 220 834
- CN-A- 113 440 591
- KR-A- 20010 068 273
- KR-A- 20110 049 618
- KR-A- 20160 040 781
- KR-A- 20210 112 570
- KR-A- 20220 044 082
- KR-A- 20220 044 082
- XIONG XINGJIANG ET AL: "Chinese herbal medicine for coronavirus disease 2019: A systematic review and meta-analysis", PHARMACOLOGICAL RESEARCH, ELSEVIER, AMSTERDAM, NL, vol. 160, 2 July 2020 (2020-07-02), XP086320456, ISSN: 1043-6618, [retrieved on 20200702], DOI: 10.1016/J.PHRS.2020.105056

## Description

### TECHNICAL FIELD

The present invention relates to a composition for preventing, ameliorating, or treating coronavirus infectious disease comprising herbal medicine mixture extract, particularly Uljatang (i.e., Otsujito) extract, as an effective component.

This work was supported by the Korea Institute of Oriental Medicine's Research and Development (R&D) Program funded by the Ministry of Science and ICT, Republic of Korea (Grant No. 1711175247).

### BACKGROUND ART

The novel Severe Acute Respiratory Syndrome Coronavirus 2 (SARS-CoV-2) is a virus with a single-stranded positive-sense RNA genome consisting of about 30,000 base pairs, surrounded by an envelope on its surface. First reported in Wuhan, China, in late December 2019, the virus rapidly spread, leading to a surge in infections worldwide. Due to its highly efficient human-to-human transmission, the World Health Organization declared it a pandemic spreading across the world and has been seeking appropriate measures to deal with it.

Typical COVID-19 patients exhibit relatively mild symptoms such as fever, cough, and chest pain. However, in some cases, the disease can progress to more severe conditions, such as difficulty breathing and pneumonia, which can lead to death.

To date, several approved treatment agents for coronavirus infections are known, including ribavirin, famciclovir, nitazoxanide, nafamostat, and chloroquine. Additionally, remdesivir (GS-5734) and favipiravir are being used to treat coronavirus infections; however, their effectiveness is limited, and the nature of nucleoside analog-type antiviral drugs tends to lead to a high occurrence rate of mutant viruses. Thus, more than two antiviral agents are often applied simultaneously. Most antiviral drugs used for coronavirus infections have an RNA polymerase inhibitory activity, but a type of coronavirus, known as Mouse Hepatitis Virus (MHV), has been discovered to show resistance to remdesivir, rendering existing antiviral drugs ineffective. Moreover, currently developed antiviral drugs have shown severe side effects, necessitating considerable caution in their application.

In addition, currently the most common treatment method is symptomatic therapy to alleviate symptoms. Therefore, there is a growing need to develop new anti-coronavirus agents that are highly effective in suppressing infections and have low toxicity for the prevention and treatment of coronavirus outbreaks.

Meanwhile, Korean Patent Publication No. 2022-0013625 discloses an anti-coronavirus composition containing *Lonicera praeflorens* extract as an effective component, and Korean Patent Publication No. 2022-0018953 discloses an antiviral composition containing an extract of fern or a fraction thereof. Further, Chinese Patent Publication No. 112 220 834 describes a traditional Chinese medecine composition for the treatment of hemorroids comprising Angelica, Bupleurum, Cimicifuga, Scutellaria, Licorice and Rhubarb. Chinese Patent Publication No. 107 802 752 describes a decoction for the treatment of recurrent oral ulcers comprising, among others, Angelica, Scutellaria barbata, Cimicifuga, Bupleurum, rhubarb and licorice. Chinese Patent Publication No. 110 433 275 describes a pharmaceutical composition for regulating the function of the body comprising Scutellaria, Rhubarb, Bupleurum, Angelica, Cimicifuga, and licorice. Chinese Patent Publication No. 113 440 591 describes several traditional Chinese medicines, in particular for the treatment of new coronary pneumonia, including SARS-Cov-2. Korean Patent Publication No. 2022 0044082 discloses a mixed extract or fractions of many different ingredients, including angelica, rhubarb, and licorice for its antiviral use against coronavirus. Finally, Xiong Xingjiang et al. (Chinese herbal medicine for coronavirus disease 2019: A systematic review and meta-analysis, Pharmacological Research, Volume 160, 2020, 105056, ISSN 1043-6618) provide a systematic review and meta-analysis of randomized controlled trials evaluating the clinical evidence for Chinese herbal medicine in the treatment of COVID-19.

However, there has been no disclosure regarding a composition for preventing, ameliorating, or treating coronavirus infectious disease comprising Uljatang extract as an effective component, as described in the present invention.

### DETAILED DESCRIPTION OF THE INVENTION

### TECHNICAL PROBLEMS TO BE SOLVED

The present invention is devised under the aforementioned circumstances, and provides compositions against coronavirus comprising Uljatang extract as an effective component. It was found in the present invention that the Uljatang extract protects cells from coronavirus infections, inhibits viral replication, and reduces the mortality rate caused by infection in an animal model of coronavirus infections. Based on those findings, the present invention is completed accordingly.

### TECHNICAL MEANS FOR SOLVING THE PROBLEMS

To achieve the object described in the above, the present text describes an antiviral composition against coronavirus comprising Uljatang extract as an effective component.

According to a first aspect, the present invention provides a pharmaceutical composition for preventing or treating coronavirus infectious disease comprising Uljatang extract as an effective component.

The present invention further provides a functional health food composition for preventing or ameliorating coronavirus infectious disease comprising Uljatang extract as an effective component.

The present invention further provides a quasi-drug composition for preventing or ameliorating coronavirus infectious disease comprising Uljatang extract as an effective component.

The present text further describes a cosmetic composition for preventing or ameliorating coronavirus infectious disease comprising Uljatang extract as an effective component.

The present invention still further provides an animal feed additive for preventing or ameliorating coronavirus infectious disease comprising Uljatang extract as an effective component.

The invention is provided in claims 1 to 6.

### ADVANTAGEOUS EFFECT OF THE INVENTION

The present invention relates to compositions for preventing, ameliorating, or treating coronavirus infectious disease comprising Uljatang extract as an effective component, and Uljatang extract is effective in protecting cells from coronavirus infections, inhibiting viral replication, and reducing the mortality rate caused by infection in an animal model of coronavirus infections.

### BRIEF DESCRIPTION OF THE DRAWINGS

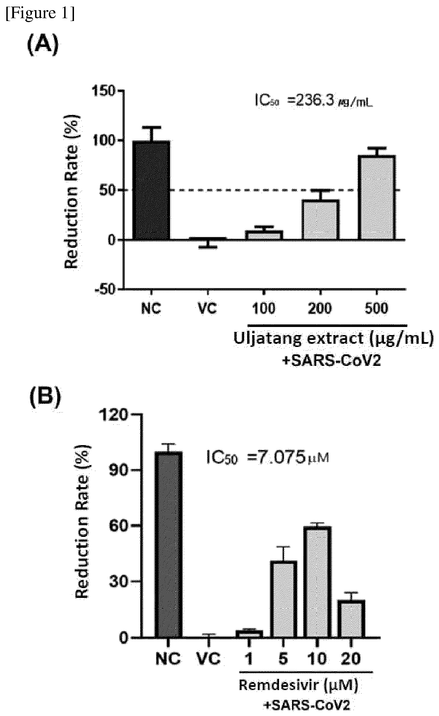

Figure 1 shows the antiviral effect of Uljatang extract against SARS-CoV2 as assessed by CPE (Cytopathic Effect) inhibition analysis. (A) shows the quantitative results of the antiviral effect of Uljatang extract at various concentrations against SARS-CoV2, while (B) shows the quantitative results obtained from the positive control, Remdesivir, against SARS-CoV2. NC represents the normal control group, and VC represents the control group treated with SARS-CoV2.

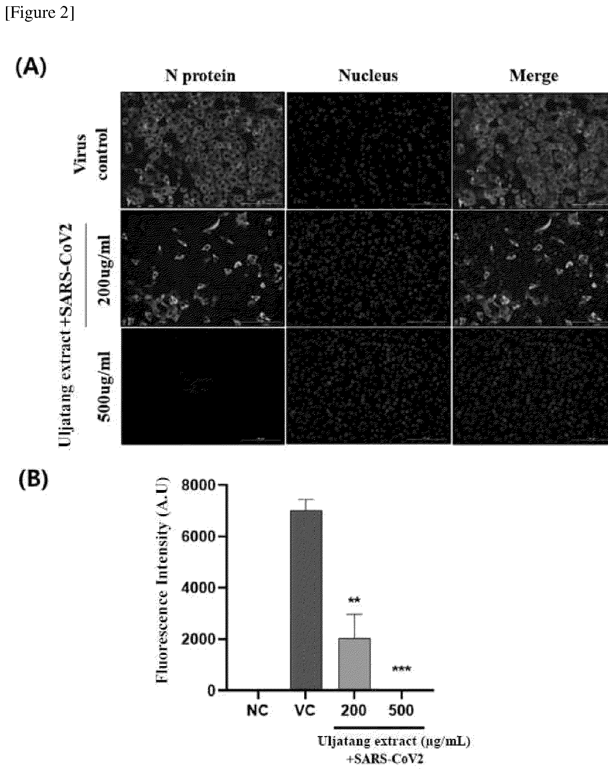

Figure 2 shows the inhibition effect of Uljatang extract on SARS-CoV2 virus infection, as observed in (A) fluorescent images and (B) quantified results of the effects. NC represents the normal control group, and VC represents the control group treated with SARS-CoV2. ** and *** indicate that the reduction in virus infection in the Uljatang extract-treated group is statistically significant compared to the SARS-CoV2-treated control group (VC), with ** denoting p<0.01 and *** denoting p<0.001.

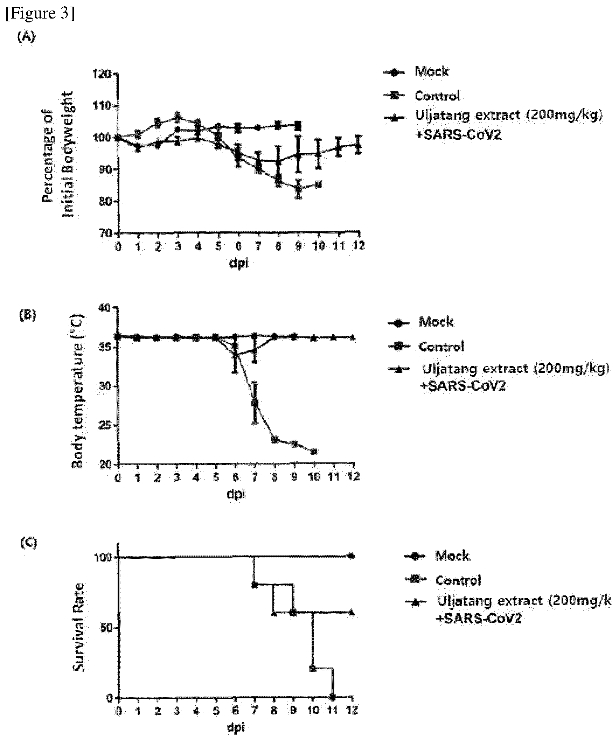

Figure 3 shows the results obtained from an animal model of the SARS-CoV2 infection, which demonstrate the effects of administration of Uljatang extract on (A) body weight, (B) body temperature, and (C) survival rate. In the figure, "Mock" represents the normal group not infected with SARS-CoV2, "Control" represents the SARS-CoV2 infection group, and "Uljatang extract (200 mg/kg)" represents the group which has been subjected to SARS-CoV2 infection followed by the administration of Uljatang extract at a dose of 200 mg/kg.

### BEST MODE(S) FOR CARRYING OUT THE INVENTION

To achieve the object of the present invention, compositions against coronavirus comprising Uljatang extract as an effective component are provided by the present invention
The coronavirus described in the present invention may be one or more selected from the group consisting of Severe Acute Respiratory Syndrome Coronavirus 2 (SARS-CoV-2), Severe Acute Respiratory Syndrome Coronavirus (SARS-CoV), and Middle East Respiratory Syndrome (MERS) Coronavirus. Preferably, it is Severe Acute Respiratory Syndrome Coronavirus 2 (SARS-CoV-2),

According to the invention, the Uljatang extract is a mixture comprising Angelica (Danggui), Bupleurum (Shiho), Scutellaria (Hwanggum), Glycyrrhiza (Gamcho), Cimicifuga rhizome (Sungma), and Rhubarb (Daehwang). The extract is a mixture which consists of 3.0 to 12.0 parts by weight of Angelica, 2.5 to 10.0 parts by weight of Bupleurum, 1.5 to 6.0 parts by weight of Scutellaria, 1.0 to 4.0 parts by weight of Glycyrrhiza, 0.75 to 3.0 parts by weight of Cimicifuga rhizome, and 0.5 to 2.0 parts by weight of Rhubarb. Preferably, the extract is a mixture which consists of 5.0 to 7.0 parts by weight of Angelica, 4.0 to 6.0 parts by weight of Bupleurum, 2.0 to 4.0 parts by weight of Scutellaria, 1.5 to 2.5 parts by weight of Glycyrrhiza, 1.0 to 2.0 parts by weight of Cimicifuga rhizome, and 0.8 to 1.2 parts by weight of Rhubarb. Most preferably, the extract is a mixture which consists of 6.0 parts by weight of Angelica, 5.0 parts by weight of Bupleurum, 3.0 parts by weight of Scutellaria, 2.0 parts by weight of Glycyrrhiza, 1.5 parts by weight of Cimicifuga rhizome, and 1.0 part by weight of Rhubarb,

The Uljatang extract of the present invention is effective in inhibiting the replication of severe acute respiratory syndrome coronavirus 2.

The present invention provides a pharmaceutical composition for preventing or treating coronavirus infectious disease comprising Uljatang extract as an effective component.

The pharmaceutical composition according to the present invention can be formulated and used in various forms, such as capsules, powders, granules, tablets, suspensions, emulsions, syrups, and aerosols for oral administration, as well as topical preparations, suppositories, and sterile injectable solutions.

The pharmaceutical composition of the present invention may further comprise a pharmaceutically acceptable carrier, excipient, or diluent.

Examples of the carrier, excipient, or diluent that may be comprised in the pharmaceutical composition of the present invention include various compounds like lactose, dextrose, sucrose, sorbitol, mannitol, xylitol, erythritol, maltitol, starch, acacia gum, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate, and mineral oil, and also a mixture thereof.

When formulating the pharmaceutical composition, common excipients, diluents, binders, moisturizers, disintegrants, and surfactants are used. Solid dosage forms for oral administration include tablets, powders, granules, and capsules, which are typically prepared by mixing the pharmaceutical composition with at least one excipient, such as starch, calcium carbonate, sucrose, lactose, or gelatin. In addition to the simple excipients, lubricants like magnesium stearate and talc are also used. Liquid dosage forms for oral administration include suspensions, solutions, emulsions, and syrups. These formulations commonly use simple diluents like water and liquid paraffin, as well as various excipients such as moisturizers, sweeteners, flavorings, and preservatives. For parenteral administration, formulations include sterile aqueous solutions, non-aqueous solvents, suspensions, emulsions, freeze-dried formulations, and suppositories. As non-aqueous solvents and suspensions, propylene glycol, polyethylene glycol, plant oils like olive oil, and injectable esters like ethyl oleate can be used. For suppositories, bases such as Witepsol, Macrogol, Twin 61, cocoa butter, laurin, and glycerogelatin can be used.

The appropriate dosage of the pharmaceutical composition of the present invention may vary depending on factors such as the formulation method, administration route, patient's age, weight, sex, medical condition, diet, administration time, administration route, excretion rate, and responsiveness.

The pharmaceutical composition of the present invention can be administered either orally or parenterally. For parenteral administration, it can be applied topically to the skin, administered intravenously, subcutaneously, intramuscularly, intraperitoneally, or transdermally.

The present invention further provides a functional health food composition for preventing or ameliorating coronavirus infectious disease comprising Uljatang extract as an effective component.

The functional health food composition can be prepared in any one formulation selected from powder, granule, pill, tablet, capsule, candy, syrup, and beverage,

When using the functional health food composition of the present invention as a food additive, the composition can be added as is or used in combination with other foods or food ingredients, and it can be applied appropriately according to conventional methods. The amount of the effective component can be adjusted according to its intended purpose (i.e., prevention or amelioration). Generally, when manufacturing food or beverages, the functional health food composition of the present invention is added in an amount not exceeding 15 parts by weight of the total ingredients, preferably not exceeding 10 parts by weight. However, for long-term consumption aimed at health purposes, this amount may be lower than the specified range. Given that there are no safety issues, the effective component may be used in amounts exceeding the specified range.

There are no specific limitations on the types of functional health food composition. Examples of foods that can incorporate the functional health food composition include meats, sausages, bread, chocolate, candies, snacks, pastries, pizza, ramen, other types of noodles, gums, dairy products including ice cream, various soups, beverages, tea drinks, alcoholic beverages, and vitamin supplements. This encompasses all health foods in the conventional sense.

In addition, the functional health food composition of the present invention can be manufactured as a food product, particularly a functional food product. This functional food includes components that are commonly added during food manufacturing, such as proteins, carbohydrates, fats, nutrients, and seasonings. For instance, when made as a beverage, the composition can include natural carbohydrates or flavoring agents in addition to the effective component. The natural carbohydrates are preferably monosaccharides (e.g., glucose, fructose), disaccharides (e.g., maltose, sucrose), oligosaccharides, polysaccharides (e.g., dextrin, cyclodextrin), or sugar alcohols (e.g., xylitol, sorbitol, erythritol). The flavoring agents that can be used may be natural flavoring agents (e.g., thaumatin, stevia extract) or synthetic flavoring agents (e.g., saccharin, aspartame).

In addition to the functional health food composition, the formulation may include various nutrients, vitamins, electrolytes, flavorings, colorants, pectin and its salts, alginic acid and its salts, organic acids, protective colloid thickeners, pH regulators, stabilizers, preservatives, glycerin, alcohol, and carbonation agents used in carbonated beverages. The proportion of these added components is not critically important, but it is generally selected within the range of 0.01 to 0.1 part by weight relative to 100 parts by weight of the functional health food composition of the present invention.

The present invention further provides a quasi-drug composition for preventing or ameliorating coronavirus infectious disease comprising Uljatang extract as an effective component.

Quasi-drug product of the present invention may include external skin preparations and personal hygiene products. For example, it may be disinfectants, shower foams, mouthwashes, wet wipes, detergent soaps, hand washes, or ointments,

When using the quasi-drug composition of the present invention as an additive in quasi-drug product, it can be added directly or used in combination with other quasi-drug products or quasi-drug components. It can be used appropriately according to conventional methods. The amount of the effective component can be suitably determined based on its intended use.

For example, the quasi-drug composition of the present invention may be manufactured in the form of typical emulsions and solubilized formulations. It can be in the form of emulsions like lotions, creams, ointments, sprays, oil gels, gels, oils, aerosols, or any other form, as long as it demonstrates effectiveness in preventing or ameliorating coronavirus infections as described in the present invention.

Additionally, the quasi-drug composition can be appropriately combined with typical ingredients used in quasi-drug products, such as fats, water, surfactants, moisturizers, low-carbon number alcohols (i.e., carbon number of 1 to 4), thickeners, chelating agents, colorants, preservatives, or fragrances, as needed.

The present text further describes a cosmetic composition for preventing or ameliorating coronavirus infectious disease comprising Uljatang extract as an effective component.

The cosmetic composition includes not only the aforementioned extract but also commonly used ingredients in cosmetic formulations, such as antioxidants, stabilizers, solubilizers, vitamins, pigments, fragrances, and carriers.

The cosmetic composition can be manufactured in any form typically produced in the industry, such as solutions, suspensions, emulsions, pastes, gels, creams, lotions, powders, soaps, surfactant-containing cleansers, oils, powder foundations, emulsion foundations, wax foundations, and sprays. More specifically, it can be formulated as toners (e.g., skin lotions), nourishing toners (e.g., milk lotions), nourishing creams, massage creams, essences, eye creams, cleansing creams, cleansing foams, cleansing waters, shampoos, masks, sprays, or powders.

If the formulation is in the form of a paste, cream, or gel, animal oils, vegetable oils, waxes, paraffin, starch, tragacanth, cellulose derivatives, polyethylene glycol, silicone, bentonite, silica, talc, or zinc oxide can be used as a carrier component.

For formulations in the form of powders or sprays, lactose, talc, silica, aluminum hydroxide, calcium silicate, or polyamide powders can be used as a carrier component. Specifically, for sprays, propellants such as chlorofluorocarbons, propane/butane, or dimethyl ether may also be included.

If the formulation is in the form of a solution or an emulsion, a solvent, a solubilizing agent, or an emulsifying agent is used as a carrier component, and examples thereof include water, ethanol, isopropanol, ethyl carbonate, ethyl acetate, benzyl alcohol, benzyl benzoate, propylene glycol, 1,3-butyl glycol oil, glycerol aliphatic esters, and fatty acid esters of polyethylene glycol or sorbitan.

If the formulation is in the form of a suspension, the carrier component that can be used include water, a liquid diluent such as ethanol or propylene glycol, a suspending agent such as ethoxylated isostearyl alcohol, polyoxyethylene sorbitol ester, and polyoxyethylene sorbitan ester, and microcrystalline cellulose, aluminum metahydroxide, bentonite, agar or tragacanth.

If the formulation is in the form of a surfactant-containing cleanser, the carrier component including aliphatic alcohol sulfates, aliphatic alcohol ether sulfates, sulfosuccinate monoesters, isethionates, imidazolinium derivatives, methyltaurate, sarcosinate, fatty acid amide ether sulfates, alkylamidobetaines, aliphatic alcohols, fatty acid glycerides, fatty acid diethanolamides, vegetable oils, lanolin derivatives, or ethoxylated glycerol fatty acid esters can be used.

The present invention still further provides an animal feed additive for preventing or ameliorating coronavirus infectious disease comprising Uljatang extract as an effective component.

The animal feed additive of the present invention is classified as a supplementary feed under feed management regulations. In the present invention, the term 'feed' refers to any natural or artificial diet, single diet, or components thereof that animals consume, ingest, and digest. The type of feed is not specifically limited, and any feed commonly used in the relevant field can be utilized. Examples of the feed include plant-based feeds such as grains, tubers, food processing by-products, algae, fiber-rich materials, pharmaceutical by-products, oils, starches, and bran or grain by-products; and animal-based feeds such as proteins, minerals, fats, mineral-based materials, single-cell proteins, animal plankton, or food waste. These feeds can be used either alone or mixed in combinations of two or more types.

Hereinbelow, the present invention is explained in greater detail in view of Preparation Example and Examples.

### EXAMPLES

### Preparation Example 1. Preparation of Uljatang extract

As for the Uljatang used in the present invention, extract of herbal mixture consisting of 6.0 g of Angelica, 5.0 g of Bupleurum, 3.0 g of Scutellaria, 2.0 g of Glycyrrhiza, 1.5 g of Cimicifuga rhizome, and 1.0 g of Rhubarb was provided by Kracie Pharma Ltd. (Japan).

### Example 1. Determination of Antivirus Effect of Uljatang Extract Based on Inhibition Analysis of CPE (cytopathic effect)

After culturing Vero E6 cells in a 96-well plate, SARS-CoV2 was inoculated at m.o.i of 0.01. Following the inoculation, Uljatang prepared according to above Preparation Example 1 was diluted with DMEM containing 2% (v/v) FBS and 1% (v/v) penicillin-streptomycin to the appropriate treatment concentrations, and then the cells were accordingly treated with the Uljatang at various concentrations. The treated cells were incubated for 3 days. After the incubation, the cell culture medium was removed, and the cells were fixed with a solution of methanol and acetone (1 : 1 v/v) at room temperature for 10 minutes. The cell culture medium was then removed, and 0.4% (w/v) crystal violet was added to each well and staining was carried out at room temperature for 10 minutes. The staining solution was then removed and the wells were dried. Then, a solution containing a mixture of methanol and acetic acid (1 : 1 v/v) was prepared, and then diluted 5-fold with distilled water. 100 µL of the solution were added to each well of the wells after drying, and crystal violet was extracted for 2 hours. Subsequently, the absorbance was measured at 550 nm.

The results indicated that, as shown in Figure 1, the treatment with the Uljatang extract of the present invention inhibited the replication of SARS-CoV2 in a concentration-dependent manner. Therefore, it was found that the Uljatang extract of the present invention exhibits excellent antiviral effects by protecting cells from SARS-CoV2 and inhibiting viral replication.

### Example 2. Determination of Effect of Inhibiting Virus Infection by Uljatang Extract

The antiviral effect of the Uljatang extract on SARS-CoV2 infection was determined by using immunofluorescence. Specifically, Vero E6 cells were seeded at 1×10⁴ cells per well in a 96-well plate and treated with Uljatang extract. Following this, SARS-CoV2, diluted 1000-fold in DMEM containing 2% (v/v) FBS and 1% (v/v) antibiotics, was added, and the cells were incubated for 24 hours. After the incubation, the cells were fixed with 4% (v/v) formaldehyde solution and then incubated with a primary antibody (N-protein) diluted 2000-fold. Subsequently, the cells were incubated with a secondary antibody diluted 200-fold. After the reaction, the cells were observed under a fluorescence microscope, and the fluorescence intensity was analyzed.

As shown in Figure 2, it was found that the Uljatang extract treatment effectively inhibits the intracellular infection caused by SARS-CoV2 virus.

### Example 3. Evaluation of efficacy of Uljatang extract in Animal Model of SARS-CoV2 Infection

To evaluate the inhibitory efficacy against SARS-CoV2 infection in hACE2 transgenic mouse model, Uljatang extract was orally administered, once a day, from 2 days before SARS-CoV2 infection until the day of death. On the day of infection (day 0), Uljatang extract of the present invention was orally administered, and 1 hour later, SARS-CoV2 was intranasally inoculated at a dose of 2,000 pfu/mouse (20 µL/mouse) for animal infection. Body weight and temperature of the animal were measured daily until the end of the experiment.

The results, as shown in Figure 3, indicated that the body weight of the Uljatang extract-treated group of the present invention increased after day 6 compared to the SARS-CoV2 infection group. Additionally, while the body temperature of the SARS-CoV2 infection group sharply decreased on day 6, the Uljatang extract-treated group of the present invention maintained a temperature similar to the normal level. The survival rate of the SARS-CoV2 infection group drastically decreased by day 10, whereas the survival rate of the Uljatang extract-treated group of the present invention remained around 60%. Therefore, it was concluded that the Uljatang extract of the present invention can effectively reduce the mortality rate in a group with severe SARS-CoV2 infection.

## Claims

1. A pharmaceutical composition for its use for preventing or treating Severe Acute Respiratory Syndrome Coronavirus 2 (SARS-CoV-2) infectious disease comprising Uljatang extract as an effective component,
wherein Uljatang is a mixture consisting of 3.0 to 12.0 parts by weight of Angelica, 2.5 to 10.0 parts by weight of Bupleurum, 1.5 to 6.0 parts by weight of Scutellaria, 1.0 to 4.0 parts by weight of Glycyrrhiza, 0.75 to 3.0 parts by weight of Cimicifuga rhizome, and 0.5 to 2.0 parts by weight of Rhubarb.

2. The pharmaceutical composition for its use according to Claim 1, wherein it further comprises a pharmaceutically acceptable carrier, excipient, or diluent in addition to the Uljatang extract.

3. A functional health food composition for its use for preventing or ameliorating Severe Acute Respiratory Syndrome Coronavirus 2 (SARS-CoV-2) infectious disease comprising Uljatang extract as an effective component,
wherein Uljatang is a mixture consisting of 3.0 to 12.0 parts by weight of Angelica, 2.5 to 10.0 parts by weight of Bupleurum, 1.5 to 6.0 parts by weight of Scutellaria, 1.0 to 4.0 parts by weight of Glycyrrhiza, 0.75 to 3.0 parts by weight of Cimicifuga rhizome, and 0.5 to 2.0 parts by weight of Rhubarb.

4. The functional health food composition for its use according to Claim 3, wherein the composition is prepared in any one formulation selected from powder, granule, pill, tablet, capsule, candy, syrup, and beverage.

5. A quasi-drug composition selected from external skin preparations and personal hygiene products for its use for preventing or ameliorating Severe Acute Respiratory Syndrome Coronavirus 2 (SARS-CoV-2) infectious disease comprising Uljatang extract as an effective component,
wherein Uljatang is a mixture consisting of 3.0 to 12.0 parts by weight of Angelica, 2.5 to 10.0 parts by weight of Bupleurum, 1.5 to 6.0 parts by weight of Scutellaria, 1.0 to 4.0 parts by weight of Glycyrrhiza, 0.75 to 3.0 parts by weight of Cimicifuga rhizome, and 0.5 to 2.0 parts by weight of Rhubarb.

6. An animal feed additive for its use for preventing or ameliorating Severe Acute Respiratory Syndrome Coronavirus 2 (SARS-CoV-2) infectious disease comprising Uljatang extract as an effective component,
wherein Uljatang is a mixture consisting of 3.0 to 12.0 parts by weight of Angelica, 2.5 to 10.0 parts by weight of Bupleurum, 1.5 to 6.0 parts by weight of Scutellaria, 1.0 to 4.0 parts by weight of Glycyrrhiza, 0.75 to 3.0 parts by weight of Cimicifuga rhizome, and 0.5 to 2.0 parts by weight of Rhubarb.

## Patentansprüche

1. Pharmazeutische Zusammensetzung für ihre Verwendung beim Vorbeugen oder Behandeln einer SARS-CoV-2 (Severe Acute Respiratory Syndrome Coronavirus 2)-Infektionskrankheit, umfassend Uljatang-Extrakt als wirksamen Bestandteil,
wobei es sich bei Uljatang um ein Gemisch handelt, das aus 3,0 bis 12,0 Gewichtsteilen *Angelica,* 2,5 bis 10,0 Gewichtsteilen *Bupleurum,* 1,5 bis 6,0 Gewichtsteilen *Scutellaria,* 1,0 bis 4,0 Gewichtsteilen *Glycyrrhiza, 0,75* bis 3,0 Gewichtsteilen *Cimicifuga-Rhizom* und 0,5 bis 2,0 Gewichtsteilen Rhabarber besteht.

2. Pharmazeutische Zusammensetzung für ihre Verwendung nach Anspruch 1, wobei sie neben dem Uljatang-Extrakt ferner ein pharmazeutisch unbedenkliches Träger-, Hilfs-oder Verdünnungsmittel umfasst.

3. Funktionale Reformkostzusammensetzung für ihre Verwendung beim Vorbeugen oder Lindern einer SARS-CoV-2(Severe Acute Respiratory Syndrome Coronavirus 2)-Infektionskrankheit, umfassend Uljatang-Extrakt als wirksamen Bestandteil,
wobei es sich bei Uljatang um ein Gemisch handelt, das aus 3,0 bis 12,0 Gewichtsteilen *Angelica,* 2,5 bis 10,0 Gewichtsteilen *Bupleurum,* 1,5 bis 6,0 Gewichtsteilen *Scutellaria,* 1,0 bis 4,0 Gewichtsteilen *Glycyrrhiza, 0,75* bis 3,0 Gewichtsteilen *Cimicifuga-Rhizom* und 0,5 bis 2,0 Gewichtsteilen Rhabarber besteht.

4. Funktionale Reformkostzusammensetzung für ihre Verwendung nach Anspruch 3, wobei die Zusammensetzung in einer aus Pulver, Granulat, Pille, Tablette, Kapsel, Bonbon, Sirup und Getränk ausgewählten Formulierung zubereitet ist.

5. Arzneistoffartige Zusammensetzung, ausgewählt aus äußerlichen Hautpräparaten und Körperpflegeprodukten, für ihre Verwendung beim Vorbeugen oder Lindern einer SARS-CoV-2(Severe Acute Respiratory Syndrome Coronavirus 2)-Infektionskrankheit, umfassend Uljatang-Extrakt als wirksamen Bestandteil,
wobei es sich bei Uljatang um ein Gemisch handelt, das aus 3,0 bis 12,0 Gewichtsteilen *Angelica,* 2,5 bis 10,0 Gewichtsteilen *Bupleurum,* 1,5 bis 6,0 Gewichtsteilen *Scutellaria,* 1,0 bis 4,0 Gewichtsteilen *Glycyrrhiza, 0,75* bis 3,0 Gewichtsteilen *Cimicifuga-Rhizom* und 0,5 bis 2,0 Gewichtsteilen Rhabarber besteht.

6. Tierfutterzusatz für seine Verwendung beim Vorbeugen oder Lindern einer SARS-CoV-2(Severe Acute Respiratory Syndrome Coronavirus 2)-Infektionskrankheit, umfassend Uljatang-Extrakt als wirksamen Bestandteil, wobei es sich bei Uljatang um ein Gemisch handelt, das aus 3,0 bis 12,0 Gewichtsteilen *Angelica,* 2,5 bis 10,0 Gewichtsteilen *Bupleurum,* 1,5 bis 6,0 Gewichtsteilen *Scutellaria,* 1,0 bis 4,0 Gewichtsteilen *Glycyrrhiza, 0,75* bis 3,0 Gewichtsteilen *Cimicifuga-Rhizom* und 0,5 bis 2,0 Gewichtsteilen Rhabarber besteht.

## Revendications

1. Composition pharmaceutique pour son utilisation pour la prévention ou le traitement d'une maladie infectieuse du syndrome respiratoire aigu sévère coronavirus 2 (SARS-CoV-2) comprenant un extrait d'Uljatang comme composant efficace,
dans laquelle Uljatang est un mélange constitué de 3,0 à 12,0 parties en poids d'Angelica, de 2,5 à 10,0 parties en poids de Bupleurum, de 1,5 à 6,0 parties en poids de Scutellaria, de 1,0 à 4,0 parties en poids de Glycyrrhiza, de 0,75 à 3,0 parties en poids de rhizome de Cimicifuga et de 0,5 à 2,0 parties en poids de rhubarbe.

2. Composition pharmaceutique pour son utilisation selon la revendication 1, dans laquelle elle comprend en outre un support, excipient ou diluant pharmaceutiquement acceptable en plus de l'extrait d'Uljatang.

3. Composition d'aliment diététique fonctionnel pour son utilisation pour prévenir ou améliorer la maladie infectieuse du syndrome respiratoire aigu sévère coronavirus 2 (SARS-CoV-2) comprenant un extrait d'Uljatang comme composant efficace,
dans laquelle Uljatang est un mélange constitué de 3,0 à 12,0 parties en poids d'Angelica, de 2,5 à 10,0 parties en poids de Bupleurum, de 1,5 à 6,0 parties en poids de Scutellaria, de 1,0 à 4,0 parties en poids de Glycyrrhiza, de 0,75 à 3,0 parties en poids de rhizome de Cimicifuga et de 0,5 à 2,0 parties en poids de rhubarbe.

4. Composition d'aliment diététique fonctionnel pour son utilisation selon la revendication 3, la composition étant préparée dans une quelconque formulation choisie parmi une poudre, un granule, une pilule, un comprimé, une capsule, un bonbon, un sirop et une boisson.

5. Composition quasi-médicamenteuse choisie parmi des préparations cutanées externes et des produits d'hygiène personnelle pour son utilisation dans la prévention ou l'amélioration d'une maladie infectieuse du syndrome respiratoire aigu sévère coronavirus 2 (SARS-CoV-2) comprenant un extrait d'Uljatang en tant que composant efficace,
dans laquelle Uljatang est un mélange constitué de 3,0 à 12,0 parties en poids d'Angelica, de 2,5 à 10,0 parties en poids de Bupleurum, de 1,5 à 6,0 parties en poids de Scutellaria, de 1,0 à 4,0 parties en poids de Glycyrrhiza, de 0,75 à 3,0 parties en poids de rhizome de Cimicifuga et de 0,5 à 2,0 parties en poids de rhubarbe.

6. Additif pour l'alimentation animale pour son utilisation pour la prévention ou l'amélioration d'une maladie infectieuse du syndrome respiratoire aigu sévère coronavirus 2 (SARS-CoV-2) comprenant un extrait d'Uljatang comme composant efficace,
dans laquelle Uljatang est un mélange constitué de 3,0 à 12,0 parties en poids d'Angelica, de 2,5 à 10,0 parties en poids de Bupleurum, de 1,5 à 6,0 parties en poids de Scutellaria, de 1,0 à 4,0 parties en poids de Glycyrrhiza, de 0,75 à 3,0 parties en poids de rhizome de Cimicifuga et de 0,5 à 2,0 parties en poids de rhubarbe.
